# EUROPEAN PATENT APPLICATION

(11) **EP 4 807 368 A1**
(43) Date of publication of application: **16.09.2026**
(21) Application number: 26162779.8
(22) Date of filing: 06.03.2026
(51) Int. Cl.: G01N 33/5754, C07K 16/18, G01N 33/68

(54) **METHOD FOR OBTAINING INFORMATION REGARDING RENAL AMYLOIDOSIS, METHOD FOR ASSISTING IN THE DETERMINATION OF RENAL AMYLOIDOSIS, AND REAGENT KIT**

(30) Priority: 07.03.2025 JP 2025036705
(71) Applicant: National University Corporation Kumamoto University, Kumamoto-shi, Kumamoto 860-8555 (JP); SYSMEX CORPORATION, Kobe-shi Hyogo 651-0073 (JP)
(72) Inventor: TASAKI, Masayoshi, Kumamoto-shi, Kumamoto, 860-8555 (JP); UEDA, Mitsuharu, Kumamoto-shi, Kumamoto, 860-8555 (JP); NAKAO, Mitsuki, Kumamoto-hi, Kumamoto, 860-8555 (JP); IWANAGA, Niina, Kobe-shi, Hyogo, 651-0073 (JP); YANAGIDA, Masatoshi, Kobe-shi, Hyogo, 651-0073 (JP); MIURA, Masahiro, Kobe-shi, Hyogo, 651-0073 (JP)
(74) Representative: De Clercq & Partners

(57) **Abstract**

[Problem] The problem is to provide a measure to enable a determination of whether a subject develops renal amyloidosis caused by proIAPP.

[Solution] The above-mentoined problem is solved by measuring proIAPP in a biological sample from a subject, and using a result of the measuring as an indicator of proIAPP-type renal amyloidosis.

## Description

### FIELD OF THE INVENTION

The present invention relates to a method for obtaining information on renal amyloidosis. The present invention relates to a method for assisting diagnosis of renal amyloidosis. The present invention relates to a reagent kit for use in these methods.

### BACKGROUND OF THE INVENTION

Renal amyloidosis is a disease caused by the deposition of amyloid, which is a fibrous insoluble protein, in the glomeruli, blood vessels, tubulointerstitium, and the like of the kidney. In patients with renal amyloidosis, renal function often declines, leading to nephrotic syndrome such as proteinuria, edema, and decreased plasma albumin. Immunoglobulin L chain, immunoglobulin H chain, serum amyloid A, and transthyretin, among others, are known as precursor proteins of renal amyloidosis. When renal amyloidosis is suspected, urine tests and blood tests are performed. However, renal biopsy is required for definitive diagnosis. In renal biopsy, the deposition of amyloid in the tissue of the kidney is confirmed, and the precursor protein is identified.

### [Prior Art]

### [Patent Document]

**[Patent Document 1]** US Patent Application Publication No. 2015/0210759

### SUMMARY OF THE INVENTION

### [Problem to be solved by the invention]

The present inventors discovered a case of renal amyloidosis with an unknown precursor protein in a renal biopsy sample from a patient showing renal function decline. As a result of searching for the precursor protein from mass spectrometry of a tissue section, which is a renal biopsy sample, the present inventors identified proislet amyloid polypeptide (proIAPP). proIAPP is a precursor protein of islet amyloid polypeptide (IAPP) (also called "amylin"). IAPP is produced when proIAPP undergoes processing. The Patent Document 1 describes that IAPP forms and deposits amyloid fibrils in the pancreatic islets of patients with type 2 diabetes, and that aberrantly processed proIAPP may become seeds for IAPP amyloidosis. However, it has not been known until now that proIAPP can be a precursor protein of renal amyloidosis. An object of the present invention is to provide a means that enables determination of whether a subject has renal amyloidosis caused by proIAPP.

### [Measure to solve the problem]

The present invention provides a method for obtaining information on renal amyloidosis, comprising a measuring step of proIAPP in a biological sample from a subject, wherein a result of the measurement is an indicator of proIAPP-type renal amyloidosis.

The present invention provides a method for assisting diagnosis of renal amyloidosis, comprising a measuring step of proIAPP in a biological sample from a subject and a determining step, based on a result of the measurement, of whether the subject has proIAPP-type renal amyloidosis.

The present invention provides a reagent kit for use in the above-mentioned method, comprising a reagent containing a substance capable of specifically binding to proIAPP.

### [Effects of the invention]

According to the present invention, it becomes possible to perform determination of whether the subject has renal amyloidosis.

### BRIEF DESCRIPTION OF THE DRAWINGS

FIG. 1 is a diagram showing an example of the appearance of a reagent kit.
FIG. 2 shows an example of a result of Congo red staining of kidney tissue collected from a patient showing renal function decline.
FIG. 3A shows a result of a fibril formation experiment of synthetic proIAPP protein.
FIG. 3B shows a result of Congo red staining of synthetic proIAPP protein after 24 hours.
FIG. 3C shows an electron micrograph of synthetic proIAPP protein after 24 hours.
FIG. 4A shows the result of immunohistochemistry staining performed on kidney tissue of a patient using antibodies against the N-terminal region and the C-terminal region of proIAPP, respectively.
FIG. 4B shows the result of the Western blot method performed on kidney tissue of a patient and synthetic proIAPP protein using an antibody against proIAPP.
FIG. 5A shows the result of detection of proIAPP in a blood sample from a patient and a healthy subject by immunoprecipitation and the Western blot method using an antibody against proIAPP.
FIG. 5B shows a result of the Western blot method performed on a blood sample from a patient and synthetic proIAPP protein using an antibody against proIAPP.
FIG. 6A is a box plot showing the concentration of proIAPP in a blood sample in a patient group and a healthy subject group.
FIG. 6B is a box plot showing the concentration of IAPP in a blood sample in a patient group and a healthy subject group.
FIG. 7 is a box plot showing the concentration of proIAPP in a blood sample in a patient group and a disease control group.

### DETAILED DESCRIPTION

In a method for obtaining information on renal amyloidosis (hereinafter also referred to as "information obtaining method"), proIAPP in a biological sample from a subject is subjected to measuring. In this method, the result of the measurement serves as an indicator of proIAPP-type renal amyloidosis. In the present specification, "proIAPP-type renal amyloidosis" refers to renal amyloidosis in which proIAPP is a precursor protein or is suspected to be so. As shown in Examples described later, when the subject has proIAPP-type renal amyloidosis, proIAPP significantly increases in the biological sample.

The subject is not particularly limited. The information obtaining method of the present embodiment can be applied to a subject group including healthy subjects, and a subject suspected of having proIAPP-type renal amyloidosis can be extracted. In another embodiment, the subject may be a person who shows a decline in renal function. Examples include patients with chronic kidney disease (CKD), patients with diabetic nephropathy, and patients with nephrotic syndrome. CKD is a disease in which the presence of kidney damage is clear and/or a state where the estimated glomerular filtration rate (eGFR) or glomerular filtration rate (GFR) is less than 60 mL/min/1.73 m² continues for 3 months or more. The presence of kidney damage is confirmed by urinalysis, blood sample tests, image diagnosis, pathological tests, and the like. The subject may be, for example, a person having a urine protein/creatinine (Cr) ratio of 0.15 g/gCr or more, a person having a urine albumin/Cr ratio of 30 mg/gCr or more, a person having a urine protein concentration of 30 mg/dL or more, a person having an eGFR of 90 mL/min/1.73 m² or less, or a person having a GFR of 90 mL/min/1.73 m² or less. Diabetic nephropathy is one of the complications of diabetes and is a kidney disease that progresses in stages. A patient with diabetic nephropathy may be, for example, a patient diagnosed with diabetes, who has a urine albumin/Cr ratio of 30 mg/gCr or more and an eGFR or GFR of 60 mL/min/1.73 m² or less. Nephrotic syndrome refers to a case where urine protein of 3.5 g/day or more (or a urine protein/creatinine ratio of 3.5 g/gCr or more in a random urine sample) continues and the serum albumin value is 3.0 g/dL or less. The information obtaining method of the present embodiment can be applied to a subject group showing a decline in renal function, and a subject suspected of having proIAPP-type renal amyloidosis can be extracted.

Examples of the biological sample include a blood sample, or tissue or cells collected by biopsy. The blood sample is blood (whole blood), plasma, or serum collected from the subject. The tissue and cells are preferably tissue and cells collected by renal biopsy. The tissue may be formalin-fixed and paraffin-embedded. In this case, it is preferable to perform deparaffinization on the paraffin-embedded tissue. Deparaffinization itself is known, and examples include treatment in which the paraffin-embedded tissue is immersed in xylene and then immersed in ethanol.

A measurement sample may be prepared from the biological sample depending on the method for measuring proIAPP described later. For example, when performing the Western blot method, a lysate of the tissue or cells or a supernatant thereof may be used. The lysate is obtained by dissolving the tissue or cells with an appropriate surfactant (for example, Triton (trademark)-X100, NP-40, etc.) or an SDS-PAGE sample buffer. When performing mass spectrometry, protein can be extracted to prepare a measurement sample. Protein may be directly extracted from the sample collected from the subject, or all or a part of the site where amyloid deposition is observed may be used as the measurement sample. The site where amyloid deposition is observed can be collected from the tissue by, for example, laser microdissection (LMD). The tissue may be stained with a dye capable of detecting amyloid deposition, such as Congo red, to identify the site to be used for the measurement sample. The collected tissue may be digested with an enzyme such as trypsin to prepare the measurement sample.

When the biological sample is liquid and contains insoluble contaminants, the contaminants may be removed by a known means such as centrifugation or filtration. When the biological sample is liquid, the biological sample may be diluted with an appropriate aqueous medium if necessary. Such an aqueous medium is not particularly limited as long as it does not hinder the measurement of proIAPP. Examples include water, physiological saline, and buffer solutions. The buffer solution is not particularly limited as long as it has a buffering action at a nearly neutral pH (for example, a pH of 6 or more and 8 or less). Such buffer solutions include, for example, Good's buffers such as HEPES, MES, and PIPES, Tris-buffered saline (TBS), and phosphate-buffered saline (PBS).

In the information obtaining method, proIAPP is subjected to measuring as a protein biomarker. The amino acid sequence of proIAPP is represented by SEQ ID No. 1. The proIAPP subjected to measuring in the information obtaining method includes not only the full-length polypeptide but also a fragment thereof. However, the fragment of proIAPP does not include IAPP. The amino acid sequence of IAPP is represented by SEQ ID No. 2. The fragment of proIAPP may be, for example, a polypeptide in which 1 or more and 10 or less amino acid sequence residues are deleted from the N-terminus and/or the C-terminus of the amino acid sequence represented by SEQ ID No. 1.

The method for measuring proIAPP may be any method that can obtain information reflecting the amount or concentration of proIAPP in the biological sample or a measurement sample prepared from the biological sample. Preferably, the method is capable of measuring proIAPP while distinguishing it from IAPP. Examples of such a measurement method include an immunological assay, mass spectrometry, and the like. Examples of the immunological assay include enzyme-linked immunosorbent assay (ELISA), immunohistochemistry, the Western blot method, and the like. The type of ELISA method is not particularly limited, and may be any of the sandwich method, the competitive method, the direct method, the indirect method, and the like. The sandwich method is preferred. The type of immunohistochemistry is not particularly limited, and may be any of the direct method, the indirect method, the sensitization method, and the like. The Western blot method may be combined with an immunoprecipitation method.

Known ionization methods can be used for mass spectrometry. Examples of such an ionization method include the electrospray ionization (ESI) method, the atmospheric pressure chemical ionization (APCI) method, the matrix-assisted laser desorption ionization (MALDI) method, and the like. A known mass spectrometer can be used for mass spectrometry. Examples of the mass spectrometer include an ion trap (IT) type mass spectrometer, a quadrupole (Q) type mass spectrometer, a time-of-flight (TOF) type mass spectrometer, a Fourier transform ion cyclotron resonance (FTICR) type mass spectrometer, an IT-TOF type mass spectrometer, a Q-TOF type mass spectrometer, a triple quadrupole (QqQ) type mass spectrometer, and the like. Preferred mass spectrometry is Liquid Chromatography-Mass Spectrometry (LC-MS) using a combination of a liquid chromatography device and a mass spectrometer, or Liquid Chromatography-Tandem Mass Spectrometry (LC-MS/MS) using a combination of a liquid chromatography device and a tandem mass spectrometer. The liquid chromatography device is not particularly limited as long as it can be connected to a mass spectrometer. An example is a high-performance liquid chromatography (HPLC) device. An example of a tandem mass spectrometer is a triple quadrupole mass spectrometer.

When measuring proIAPP by mass spectrometry, proIAPP in the biological sample or measurement sample to which an internal standard substance has been added may be subjected to measuring as necessary. In this case, information reflecting the amount or concentration of proIAPP can be obtained based on a measurement result of the internal standard substance. The internal standard substance may be, for example, recombinant or synthetic proIAPP protein labeled with a stable isotope such as ¹³C or ¹⁵N.

In the immunological assay, the information reflecting the amount or concentration of proIAPP is preferably an optical indicator that is visually recognizable or machine-measurable. Examples of the optical indicator include luminescence intensity, fluorescence intensity, absorbance, turbidity, color development intensity, staining intensity, and staining shade. In mass spectrometry, the information reflecting the amount or concentration of proIAPP is preferably an indicator obtained from a peak corresponding to proIAPP displayed on a mass spectrum. Examples of the indicator obtained from the peak include the height, area, and width of the peak. The peak may be displayed by either relative intensity or absolute intensity.

Information reflecting the amount or concentration of proIAPP can be shown qualitatively, quantitatively, or semi-quantitatively. Qualitative information is information indicating the presence or absence of proIAPP. Quantitative information is numerical information such as numerical values obtained by a measuring instrument (hereinafter also referred to as "raw data") and values calculated from the numerical values. When proIAPP is subjected to measuring by immunohistochemistry and the Western blot method, quantitative information can be obtained by quantifying the staining intensity and band intensity from the image of the immunostained tissue and the image of the band in the Western blot method. Quantification of the staining intensity and band intensity can be performed by a known image analysis software such as Image J. Examples of the value calculated from the raw data include a value obtained by subtracting a value of a negative control sample or a background value from the raw data. The value of the amount or concentration of proIAPP can be determined based on quantitative information. Semi-quantitative information is information showing the amount or concentration of proIAPP in stages using words, numbers (indicating classes), colors, or the like. For example, words such as "below the limit of detection," "low," "medium," or "high" may be used.

The result of the measurement of proIAPP includes values, information, and a combination thereof obtained by measuring proIAPP. Preferably, the result of the measurement of proIAPP is quantitative information reflecting the amount or concentration of proIAPP and/or a value of the amount or concentration of proIAPP determined based on the quantitative information. Hereinafter, the quantitative information reflecting the amount or concentration of proIAPP and/or the value of the amount or concentration of proIAPP are also referred to as a "measured value of proIAPP".

When proIAPP is subjected to measuring by an immunological assay, a substance capable of specifically binding to proIAPP is used. Examples of the substance capable of specifically binding to proIAPP include an antibody, an aptamer, and the like. An antibody is preferable. In the present specification, "antibody" includes not only the immunoglobulin form but also antibody fragments. Examples of the antibody fragments include Fab, F(ab') 2, Fab', Fv, Fd, domain antibody (dAb), single-chain antibody (scFv), and diabody. The antibody may be either a monoclonal antibody or a polyclonal antibody. An antibody against proIAPP (hereinafter also referred to as "anti-proIAPP antibody") itself is known and generally available.

The anti-proIAPP antibody is preferably an antibody that specifically binds to proIAPP and does not substantially bind to IAPP. In the present specification, "substantially does not bind to IAPP" includes that the antibody does not bind to IAPP and that the antibody binds to IAPP to the extent that it does not affect the measurement result of proIAPP. The antibody that specifically binds to proIAPP and substantially does not bind to IAPP is, for example, an antibody that specifically binds to an epitope present in the region of position 1 to 13 or position 47 to 67, preferably the region of position 1 to 11 or position 49 to 67, of the amino acid sequence represented by SEQ ID No. 1, when the epitope to which the antibody binds includes at least 3 amino acid sequence residues.

When proIAPP in the biological sample is subjected to measuring by the sandwich ELISA method, an antibody for capturing proIAPP (hereinafter also referred to as a "capture antibody") and an antibody for detecting proIAPP (hereinafter also referred to as a "detection antibody") are used. At least one of the capture antibody and the detection antibody may be an anti-proIAPP antibody. For example, both the capture antibody and the detection antibody may be anti-proIAPP antibodies. When both the capture antibody and the detection antibody, which are anti-proIAPP antibodies, are monoclonal antibodies, it is preferable that their epitopes are different from each other. Alternatively, the capture antibody may be an anti-proIAPP antibody, and the detection antibody may be an antibody against IAPP (hereinafter also referred to as "anti-IAPP antibody"). Further, the capture antibody may be an anti-IAPP antibody, and the detection antibody may be an anti-proIAPP antibody. Here, the anti-IAPP antibody is an antibody that specifically binds to an epitope present in the region of position 12 to 48 of SEQ ID No. 1. Therefore, the anti-IAPP antibody binds to proIAPP as well as to IAPP. The anti-IAPP antibody itself is known and generally available.

As an example of the measurement of proIAPP, measurement by the sandwich ELISA method is described below. First, a complex containing proIAPP, a capture antibody, and a detection antibody is formed on a solid phase. The complex can be formed by mixing the biological sample, the capture antibody, and the detection antibody. The complex formation step is preferably carried out in a solution containing proIAPP, the capture antibody, and the detection antibody. The above complex can be formed on the solid phase by bringing the solution containing the formed complex into contact with a solid phase capable of immobilizing the capture antibody. Alternatively, a solid phase on which the capture antibody has been immobilized in advance may be used. In this case, the above complex can be formed on the solid phase by bringing the solid phase on which the capture antibody is immobilized into contact with the biological sample and the detection antibody.

The solid phase may be an insoluble carrier capable of immobilizing the capture antibody. The mode of immobilization of the capture antibody to the solid phase is not particularly limited. For example, the capture antibody and the solid phase may be directly bound, or the capture antibody and the solid phase may be indirectly bound via another substance. Examples of direct binding include physical adsorption. Examples of indirect binding include binding via a combination of biotins and avidins. By modifying the capture antibody with biotins in advance and binding avidins to the solid phase in advance, the capture antibody and the solid phase can be indirectly bound via the binding between the biotins and the avidins.

In the present specification, "biotins" includes biotin and its analogs. Examples of biotin analogs include desthiobiotin and biocytin. In the present specification, "avidins" includes avidin and its analogs. Examples of avidin analogs include streptavidin, Hypsizygus marmoreus-derived avidin-like protein (Tamavidin (registered trademark)), bradavidin, and rhizavidin.

The material of the solid phase is not particularly limited and can be selected from, for example, organic polymer compounds, inorganic compounds, biopolymers, and the like. Examples of organic polymer compounds include latex, polystyrene, and polypropylene. Examples of inorganic compounds include magnetic substances (iron oxide, chromium oxide, and ferrite, etc.), silica, alumina, and glass. Examples of biopolymers include insoluble agarose, insoluble dextran, gelatin, and cellulose. Two or more of these may be used in combination. The shape of the solid phase is not particularly limited, and examples include particles, membranes, microplates, microtubes, and test tubes. Among them, particles are preferable, and magnetic particles are particularly preferable.

B/F separation (Bound/Free separation), which removes unreacted free components not forming a complex, may be performed between the complex formation step and a complex detection step described later. Unreacted free components refer to components that do not constitute the complex. Examples include the capture antibody and the detection antibody that did not bind to proIAPP. The means for B/F separation (Bound/Free separation) is not particularly limited. When the solid phase is particles, only the solid phase that has captured the complex is collected by centrifugation. This allows for B/F separation (Bound/Free separation). When the solid phase is a container such as a microplate or a microtube, B/F separation (Bound/Free separation) can be performed by removing the liquid containing unreacted free components. When the solid phase is magnetic particles, the liquid containing unreacted free components is suctioned and removed by a nozzle while the magnetic particles are magnetically constrained by a magnet. This allows for B/F separation (Bound/Free separation). B/F separation (Bound/Free separation) using magnetic particles and a magnet is preferable from the viewpoint of automation. After removing the unreacted free components, the solid phase that has captured the complex may be washed with an appropriate aqueous medium such as PBS.

proIAPP in the biological sample can be subjected to measuring by detecting the complex formed on the solid phase by a known method. The detection of the complex is preferably performed in a solution. When an antibody labeled with a labeling substance is used as the detection antibody, a signal is generated by the labeling substance from the detection antibody contained in the complex on the solid phase. The complex on the solid phase is detected by detecting the signal. Since this complex includes proIAPP captured from the biological sample by the capture antibody, the detection of the complex results in the measurement of proIAPP in the biological sample. When a labeled secondary antibody against the detection antibody is used, proIAPP in the biological sample can be subjected to measuring in the same manner.

As a further example of the method for measuring proIAPP using an antibody, the immune complex transfer method described in JP-A-H01-254868 can also be used.

The labeling substance is not particularly limited as long as a detectable signal is generated. For example, it may be a substance that itself generates a signal (hereinafter also referred to as a "signal generating substance"), or a substance that catalyzes the reaction of another substance to generate a signal. Examples of the signal generating substance include a fluorescent substance, a radioisotope, and the like. Examples of the substance that catalyzes the reaction of another substance to generate a detectable signal include an enzyme. Examples of the enzyme include alkaline phosphatase, peroxidase, β-galactosidase, and luciferase. Examples of fluorescent substances include fluorescent dyes such as fluorescein isothiocyanate (FITC), rhodamine, and Alexa Fluor (registered trademark), and fluorescent proteins such as GFP. Examples of the radioisotope include ¹²⁵I,¹⁴C, and ³²P. Among them, an enzyme is preferable as the labeling substance, and alkaline phosphatase and peroxidase are particularly preferable.

The method for detecting a signal itself is known in the technical field. A measurement method corresponding to the type of signal derived from the above-mentioned labeling substance can be appropriately selected. For example, when the labeling substance is an enzyme, a signal such as light or color generated by reacting with a substrate for the enzyme is measured. Known equipment such as a spectrophotometer, a microplate reader, an X-ray film, an imaging plate, and a reader thereof can be used for the measurement of the signal.

The substrate for the enzyme can be appropriately selected from known substrates according to the type of the enzyme. For example, when alkaline phosphatase is used, the following substances are used as substrates, for example.
- CDP-Star (registered trademark) (disodium 4-chloro-3-(methoxyspiro[1,2-dioxetane-3,2'-(5'-chloro)tricyclo[3.3.1.1^{3,7}]decan]-4-yl)phenyl phosphate)
- CSPD (registered trademark) (disodium 3-(4-methoxyspiro[1,2-dioxetane-3,2'-(5'-chloro)tricyclo[3.3.1.1^{3,7}]decan]-4-yl)phenyl phosphate)
- 5-bromo-4-chloro-3-indolyl phosphate (BCIP)
- Disodium 5-bromo-6-chloro-indolyl phosphate
- p-nitrophenyl phosphate

Furthermore, when peroxidase is used, the following substances are used as substrates, for example.
- Luminol and its derivatives
- 2,2'-azinobis(3-ethylbenzothiazoline-6-sulfonic acid) ammonium salt (ABTS)
- 1,2-phenylenediamine (OPD)
- 3,3',5,5'-tetramethylbenzidine (TMB)

When the labeling substance is a radioisotope, radiation as a signal can be measured using known equipment such as a scintillation counter, an X-ray film, an imaging plate, and a reader thereof. When the labeling substance is a fluorescent substance, fluorescence as a signal can be measured using known equipment such as a microplate reader, a confocal laser microscope, and a fluorescence microscope. The excitation wavelength and the fluorescence wavelength can be appropriately determined according to the type of fluorescent substance used.

The signal detection result can be used as the measurement result of proIAPP. For example, when quantifying the signal intensity, the measurement value of the signal intensity itself or a value obtained from the measurement value can be used as the measurement result of proIAPP. Examples of the value obtained from the measurement value of the signal intensity include a value obtained by subtracting the measurement value of a negative control sample or a background value from the measurement value of the signal intensity. The value of the amount or concentration of proIAPP may be determined by applying the measurement value of the signal intensity to a calibration curve. A negative control sample can be appropriately selected. Examples include a biological sample obtained from a healthy subject.

proIAPP contained in the biological sample can be subjected to measuring by the sandwich ELISA method using a capture antibody immobilized on magnetic particles and a detection antibody labeled with a labeling substance. In this case, the measurement may be performed using a commercially available fully automated immunoassay device. Examples of such a fully automated immunoassay device include the HISCL (registered trademark) series from Sysmex Corporation.

As described above, the result of the measurement of proIAPP serves as an indicator of the presence of proIAPP-type renal amyloidosis. For example, as shown in the Examples, the concentration of proIAPP in the biological sample in the patient group with proIAPP-type renal amyloidosis was significantly higher than in the healthy subject group and the disease control group. Therefore, the result of the measurement of proIAPP can be obtained as information on renal amyloidosis. For example, when the measured value of proIAPP is equal to or greater than a threshold, the measured value of proIAPP suggests that the subject has proIAPP-type renal amyloidosis. When the measured value of proIAPP is less than the threshold, the measured value of proIAPP suggests that the subject does not have proIAPP-type renal amyloidosis. Alternatively, when the measured value of proIAPP is higher than the threshold, the measured value of proIAPP suggests that the subject has proIAPP-type renal amyloidosis. When the measured value of proIAPP is equal to or less than the threshold, the measured value of proIAPP suggests that the subject does not have proIAPP-type renal amyloidosis.

The threshold for the measured value of proIAPP is not particularly limited and can be set appropriately. For example, the threshold may be set as follows. First, a biological sample is collected from a patient group with proIAPP-type renal amyloidosis and a healthy subject group, and proIAPP is subjected to measuring to obtain the measured value of proIAPP. Then, a value that can distinguish the patient group and the healthy subject group with the highest accuracy is determined, and this value is set as the threshold. In setting the threshold, it is preferable to consider sensitivity, specificity, positive predictive value, and negative predictive value.

Another embodiment of the present invention relates to a method for assisting diagnosis of renal amyloidosis (hereinafter also referred to as "diagnosis assisting method"). This diagnosis assisting method includes a measuring step of proIAPP in a biological sample from a subject, and a determining step, based on a result of the measurement, of whether the subject has proIAPP-type renal amyloidosis. The measuring step of proIAPP in the biological sample from the subject is as described above. In the determining step, the measured value of proIAPP is preferably compared with a threshold, and the determining step is performed based on the comparison result. For example, when the measured value of proIAPP is equal to or greater than the threshold, the subject may be determined to have proIAPP-type renal amyloidosis. When the measured value of proIAPP is less than the threshold, the subject may be determined not to have proIAPP-type renal amyloidosis. Alternatively, when the measured value of proIAPP is higher than the threshold, the subject may be determined to have proIAPP-type renal amyloidosis. When the measured value of proIAPP is equal to or less than the threshold, the subject may be determined not to have proIAPP-type renal amyloidosis. Details of the subject, the biological sample, the measurement of proIAPP, and the threshold are as described above.

The present invention includes a method for treating renal amyloidosis. The treatment method according to the present invention includes a step of performing renal amyloidosis treatment on the subject suggested to have proIAPP-type renal amyloidosis. Examples of renal amyloidosis treatment include drug therapy, hematopoietic stem cell transplantation, exercise therapy, and diet therapy, and these treatment methods can be adopted alone or in combination. For example, drug therapy includes the administration of drugs such as antitumor agents (bortezomib, thalidomide, lenalidomide, melphalan, etc.), anti-inflammatory agents (dexamethasone, etc.), immunosuppressive agents (tocilizumab, etc.), and disease-modifying substances (siRNA, etc.). These drugs can be used alone or in combination.

The present invention also includes a reagent kit (hereinafter also referred to as "reagent kit") used in the method for obtaining information on renal amyloidosis or the method for assisting diagnosis of renal amyloidosis. The reagent kit includes a reagent containing a substance capable of specifically binding to proIAPP. Details of the substance capable of specifically binding to proIAPP are as described above.

The reagent containing the substance capable of specifically binding to proIAPP is preferably a reagent containing an antibody against proIAPP. More preferably, it is a combination of a reagent containing an antibody for capturing proIAPP (capture antibody) and a reagent containing an antibody for detecting proIAPP (detection antibody). The reagent kit includes a reagent containing a capture antibody and a reagent containing a detection antibody. The detection antibody may be labeled with a labeling substance. When the labeling substance is an enzyme, the reagent kit may contain a substrate for the enzyme. Details of the capture antibody, detection antibody, labeling substance, and substrate are as described above. The form of the capture antibody, detection antibody, labeling substance, and substrate is not particularly limited, and may be a solid (for example, powder, crystal, lyophilized product, etc.) or a liquid (for example, solution, suspension, emulsion, etc.).

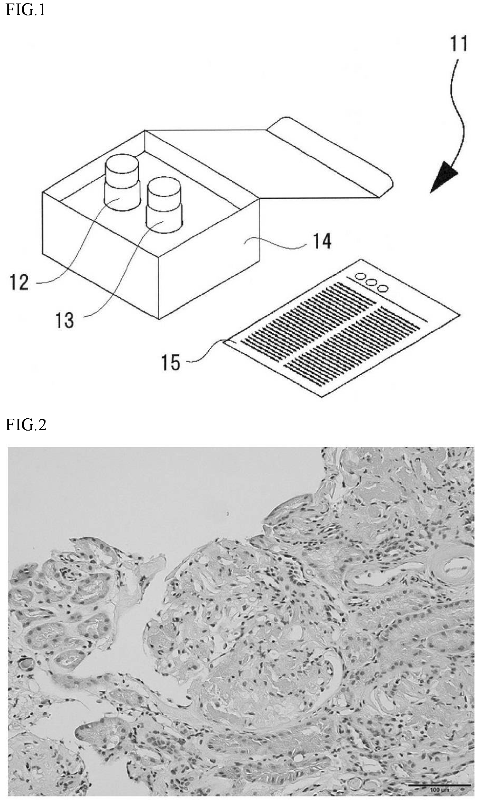

The reagent kit can be provided to the user in a state where containers accommodating the reagents are packaged in a box. A package insert may be enclosed in the box. The package insert may describe the configuration of the reagent kit, the method of use, the relationship between the measurement result obtained by the reagent kit and the patient's pathological condition, and the like. An example of such a reagent kit is shown in the figure. The reagent kit shown in FIG. 1 includes a reagent containing a capture antibody and a reagent containing a detection antibody, but the present invention is not limited to this example. Referring to FIG. 1, 11 indicates the reagent kit, 12 indicates a first container accommodating the reagent containing the capture antibody, 13 indicates a second container accommodating the reagent containing the detection antibody, 14 indicates a packaging box, and 15 indicates a package insert. In this example, the reagent kit may further include a solid phase for immobilizing the capture antibody. Details of the solid phase are as described above.

The reagent kit may further include a calibrator for proIAPP quantification. The calibrator for proIAPP quantification is, for example, a buffer solution containing recombinant or synthetic proIAPP protein at a predetermined concentration. There may be one or a plurality of buffer solutions containing proIAPP at a predetermined concentration. When there are a plurality of buffer solutions containing proIAPP at a predetermined concentration, it is preferable to prepare each buffer solution so that the proIAPP concentrations are different from each other by serial dilution or the like. The calibrator for proIAPP quantification may further include a buffer solution that does not contain proIAPP (negative control).

The present invention includes the use of an anti-proIAPP antibody for manufacturing the above-mentioned reagent kit. Details of the reagent are as described above.

Hereinafter, the present invention will be described in detail with reference to Examples, but the present invention is not limited to these Examples.

### [EXAMPLES]

### Reference Example 1: Congo red staining of kidney tissue

The specimens were formalin-fixed tissue sections of kidney tissue collected for renal biopsy from patients (5 cases) who showed renal function decline. These formalin-fixed tissue sections were deparaffinized by a conventional method and then stained with a Congo red staining solution for 90 minutes. The stained tissue sections were washed with water and subjected to nuclear staining with a hematoxylin solution for 1 minute. After washing the stained tissue sections with water, they were mounted and observed under a microscope. An example of the Congo red stained kidney tissue is shown in FIG. 2. Amyloid deposition was observed in all kidney tissues, and the patients were determined to be positive for amyloidosis. However, none of the cases corresponded to any known amyloidosis, and they were amyloidosis of unknown etiology.

### Example 1: Mass Spectrometry

Mass spectrometry was performed on the specimens (formalin-fixed tissue sections or frozen tissue sections) obtained for renal biopsy from the patients (5 cases) in Reference Example 1 to identify the precursor protein of amyloidosis.

### (1) Mass Spectrometry

Formalin-fixed tissue sections or frozen tissue sections were mounted on an LMD membrane slide (Leica Microsystems) and stained with Congo red solution. When using formalin-fixed tissue sections, they were deparaffinized by a conventional method and then stained with Congo red solution. A site where amyloid deposition was observed in the stained tissue section was collected by LMD to obtain a sample. The sample was heated in a buffer (40µL) containing 1 mM EDTA, 10 mM Tris, and 0.002% Zwittergent 3-16 (Calbiochem) at 98°C for 90 minutes. After sonicating the heated sample for 60 minutes, 1.0µg/µL trypsin solution (1.5µL) was added, and the sample was incubated at 37°C overnight. Thereby, a sample solution in which protein in the sample was fragmented into peptides was obtained. 2µL of 100 mM dithiothreitol solution was added to the sample solution, and reduction treatment was performed by heating at 95°C for 5 minutes. The reduced sample solution was dried using a centrifugal concentrator, and then redissolved by adding (40µL) of MS-grade water containing 2% acetonitrile and 0.1% trifluoroacetic acid. Peptides were eluted by a gradient of 0.1% formic acid solution and 80% acetonitrile/0.1% formic acid solution. Mass spectrometry was performed by a Vanquish Neo UHPLC system coupled with a Q-Exactive Plus Orbitrap mass spectrometer (Thermo Fisher Scientific). Protein in the sample was identified by Sequest HT in Proteome Discoverer (Thermo Fisher Scientific) software using the Homo sapiens database. The conditions for data analysis were as follows: false discovery rate: 1%, Max. missed cleavage: 2, Min. peptide length: 6, Max. peptide length: 150, Precursor mass tolerance: 10 ppm, Fragment mass tolerance: 0.02 Da. Dynamic modifications: Oxidation (+15.995 Da; Met), methylol (+30.011 Da; Lys, Trp, Tyr), Thiazolidine (+12.000 Da; Phe, His, Lys, Arg, Trp, Tyr).

### (2) Results

As an example of the analysis results, Tables 1-1 and 1-2 show lists of proteins detected from the samples of two patients among the patients in Reference Example 1. The relative abundance of each protein was calculated using the normalized spectral abundance factor.

**[Table 1-1]**

| Accession | Protein | Detected peptides (n) | Relative value |
|---|---|---|---|
| P02649 | Apolipoprotein E | 150 | 0.0285 |
| P02743 | Serum amyloid P-component | 93 | 0.0251 |
| P60709 | Actin, cytoplasmic 1 | 137 | 0.0220 |
| * P10997 | Islet amyloid polypeptide | 22 | 0.0198 |
| P68871 | Hemoglobin subunit beta | 42 | 0.0172 |
| P68133 | Actin, alpha skeletal muscle | 102 | 0.0163 |
| P08670 | Vimentin | 126 | 0.0163 |
| P62736 | Actin, aortic smooth muscle | 100 | 0.0160 |
| P62805 | Histone H4 | 23 | 0.0134 |
| P02768 | Albumin | 129 | 0.0127 |

**[Table 1-2]**

| Accession | Protein | Detected peptides (n) | Relative value |
|---|---|---|---|
| P02649 | Apolipoprotein E | 135 | 0.0248 |
| P02743 | Serum amyloid P-component | 85 | 0.0222 |
| P60709 | Actin, cytoplasmic 1 | 132 | 0.0205 |
| * P10997 | Islet amyloid polypeptide | 21 | 0.0182 |
| P68871 | Hemoglobin subunit beta | 42 | 0.0166 |
| P68133 | Actin, alpha skeletal muscle | 107 | 0.0165 |
| P62736 | Actin, aortic smooth muscle | 104 | 0.0160 |
| P62805 | Histone H4 | 27 | 0.0152 |
| P02768 | Albumin | 152 | 0.0145 |
| P08670 | Vimentin | 100 | 0.0125 |

As shown in Tables 1-1 and 1-2, proteins commonly found in kidney tissue and proteins already known to coexist with amyloid fibrils were detected from the patient samples, with the exception of islet amyloid polypeptide (IAPP) marked with an asterisk. The present inventors focused on IAPP. Here, the amino acid sequence of proIAPP including the signal peptide region (SEQ ID No. 3) was registered in the database as the amino acid sequence of IAPP. Further, when confirming the data of fragments identified as IAPP by mass spectrometry, a polypeptide longer than the amino acid sequence of IAPP was detected. This polypeptide was found to be proIAPP, a precursor protein of IAPP. The present inventors presumed that proIAPP is the precursor protein of the amyloidosis in the patients. In creating the above list, the amino acid sequence (SEQ ID No. 1) obtained by deleting the signal peptide region (positions 1 to 22) of proIAPP from the database amino acid sequence (SEQ ID No. 3) was used in order to increase the detection rate of the N-terminal region of proIAPP.

### Reference Example 2: Experiment of fibril formation of proIAPP

In order to examine whether proIAPP can cause amyloidosis, it was confirmed whether proIAPP forms amyloid fibrils in vitro.

### (1) Fibril Formation Experiment

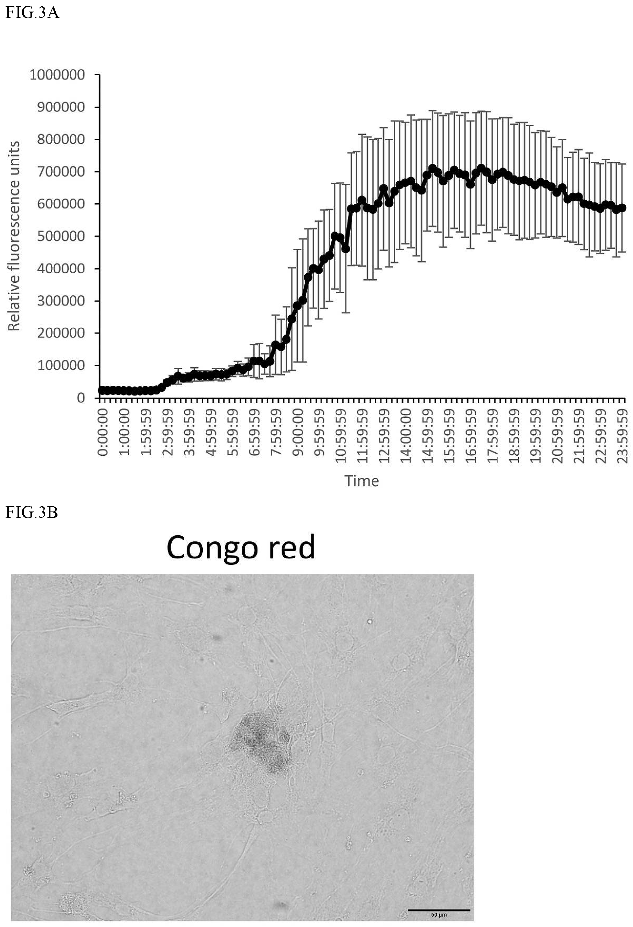

The proIAPP protein consisting of the amino acid sequence represented by SEQ ID No. 1 was purchased from Hokkaido System Science Co., Ltd. Hereinafter, the obtained proIAPP protein is also referred to as "synthetic proIAPP protein". The synthetic proIAPP protein was dissolved in dimethyl sulfoxide to prepare a 2 mM stock solution and stored at -80°C until use in experiments. The stock solution was diluted with PBS to prepare a 50µM proIAPP solution (100µL). Thioflavin T solution was added to this proIAPP solution so that the final concentration was 10µM. The obtained solution was dispensed into wells of a 96-well black plate (Greiner Bio-one), and the plate was sealed with a transparent film. The sealed plate was shaken by an orbital shaker at 37°C and 448 rpm (Low mode). The plate was shaken for 5 minutes at 10-minute intervals. The plate was set on a microplate reader SpectraMax iD3 (MOLECULAR DEVICES), and the fluorescence intensity was measured every 15 minutes. In the measurement, the excitation wavelength (ex) was 460 nm, and the fluorescence wavelength (em) was 510 nm. The results are shown in FIG. 3A.

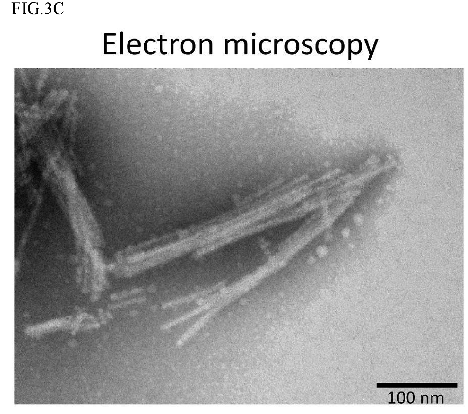

From the stock solution, a 100µM proIAPP solution was shaken at 37°C for 24 hours in the same manner as above. After culturing glomotel cells in Lab-Tek chamber slides (Thermo Fisher Scientific) for 24 hours, the proIAPP solution shaken for 24 hours was added. Glomotel cells were used as a scaffold for amyloid adhesion. After further standing in 5%CO₂ at 37°C for 24 hours, the supernatant was removed, washed with PBS, and fixed with 4% paraformaldehyde solution for 15 minutes. After washing, the sections were stained with Congo red staining solution for 60 minutes. After washing with PBS, ProLong^{™} Gold Antifade Mountant (Thermo Fisher Scientific) was dropped, and the sections were mounted with a coverslip and observed under a microscope. The results are shown in FIG. 3B. The stock solution was diluted with PBS, and a 50µM proIAPP solution was shaken at 37°C for 24 hours in the same manner as above. The prepared proIAPP solution was dropped onto a copper grid and stained with 2% phosphotungstic acid solution for 30 seconds. The prepared specimen was observed with a transmission electron microscope. The results are shown in FIG. 3C.

### (2) Results

As shown in FIG. 3A, the fluorescence intensity increased over time from the start of the measurement. Since Thioflavin T is a compound that binds to amyloid fibrils, it was suggested that proIAPP formed fibrils. As can be seen from FIG. 3B, proIAPP shaken for 24 hours was stained by Congo red. Furthermore, as shown in FIG. 3C, proIAPP formed a fibrillar structure. These results also suggested that proIAPP formed fibrils. Therefore, it was suggested that proIAPP is a fibril-forming protein and can cause amyloidosis.

### Example 2: Detection of proIAPP by immunohistochemistry (IHC) and Western blotting (WB) method

It was examined whether proIAPP could be detected by performing IHC staining and the WB method on specimens (formalin-fixed tissue sections) obtained by renal biopsy from the patients (3 cases) in Reference Example 1.

### (1) IHC Staining

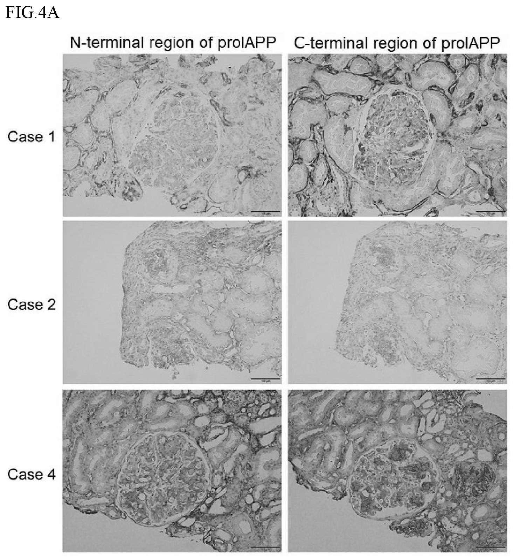

Rabbit-derived antibodies against the N-terminal region (positions 23 to 35 of SEQ ID No. 3) and the C-terminal region (positions 74 to 89 of SEQ ID No. 3) of proIAPP, respectively, were prepared by commissioning COSMO BIO CO., LTD. Hereinafter, each antibody is also referred to as "anti-proIAPP N-terminal antibody" and "anti-proIAPP C-terminal antibody". IHC staining was performed using the Autostainer Link 48 (Agilent). After deparaffinizing the tissue sections, antigen retrieval was performed with 100% formic acid for 1 minute. Non-specific signals were blocked with EnVision FLEX peroxidase-blocking reagent (Agilent). As primary antibodies, the anti-proIAPP N-terminal antibody (1:500 dilution) and the anti-proIAPP C-terminal antibody (1:500 dilution) were used. As a secondary antibody, EnVision FLEX/HRP (Agilent) was used. The signal was visualized with EnVision FLEX DAB+CHROMOGEN solution (Agilent) diluted with EnVision FLEX substrate buffer (Agilent), and the nuclei were stained with hematoxylin. The results are shown in FIG. 4A.

### (2) WB Method

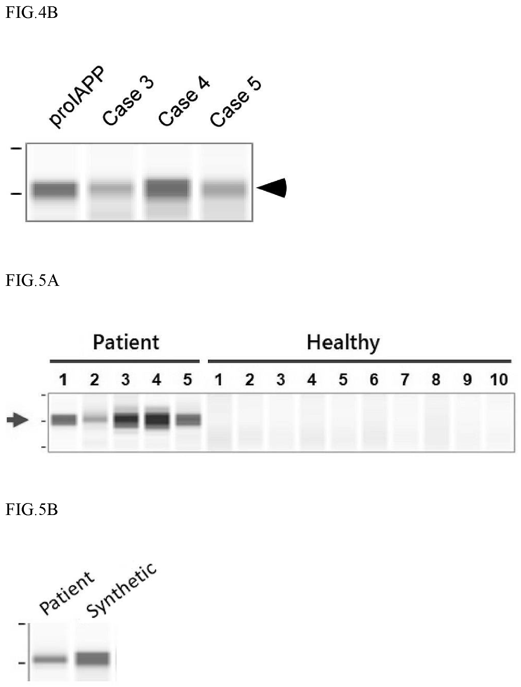

In the same manner as in Example 1, the amyloid deposition site was collected from the tissue section by LMD. To the collected site, a buffer (40µL) containing 1 mM EDTA, 10 mM Tris, and 0.002% Zwittergent 3-16 (Calbiochem) and 2×sample buffer (40µL, with a final concentration of 5% 2-mercaptoethanol) were added, and the mixture was heated at 98°C for 90 minutes. Thereafter, sonication was performed for 60 minutes to obtain a sample for electrophoresis. In addition, the synthetic proIAPP protein was diluted with PBS and prepared as a control sample for electrophoresis. WB was performed using the simple Western system Wes (Protein Simple). As the primary antibody, a mouse monoclonal anti-IAPP antibody against proIAPP (positions 40 to 89 of SEQ ID No. 3) (sc-377530; Santa Cruz Biotechnology; hereinafter also referred to as "anti-amylin antibody") (1:250 dilution) was used, and as the secondary antibody, an Anti-Mouse Secondary Antibody (Protein Simple) was used. An example of the results is shown in FIG. 4B.

### (3) Results

As shown in FIG. 4A, proIAPP was detected by IHC staining in the kidney tissue sections of all cases. As shown in FIG. 4B, a band to which the anti-proIAPP antibody bound was observed at the same position as the synthetic proIAPP protein in the kidney tissue. Therefore, it was shown that proIAPP in the kidney tissue can be detected by IHC staining and the WB method.

### Example 3: Detection of proIAPP in blood sample by immunoprecipitation and WB method

It was examined whether proIAPP in the plasma and serum of the patients in Reference Example 1 could be detected by immunoprecipitation and the WB method. For comparison, plasma from healthy subjects was used.

### (1) Immunoprecipitation and WB Method

The Dynabeads^{™} Protein G Immunoprecipitation Kit (Invitrogen) was used for immunoprecipitation. Dynabeads^{™} magnetic particles (50µL) were added to a 1.5 mL tube. After immobilizing the magnetic particles with a magnet, the supernatant was removed. 2µg of the anti-proIAPP C-terminal antibody was diluted with Ab Binding and Washing Buffer (200µL). The obtained antibody solution was added to the magnetic particles and incubated for 10 minutes at room temperature with rotation. The magnetic particles were washed 3 times with Ab Binding and Washing Buffer (200µL), and the supernatant was removed. Plasma or serum (200µL) was added to the magnetic particles and incubated for 30 minutes at room temperature with rotation. After washing the magnetic particles 3 times with Ab Binding and Washing Buffer (200µL), Ab Binding and Washing Buffer (100µL) was added to the magnetic particles. After transferring the magnetic particles to a new tube, the supernatant was removed. Water (20µL) and 2×sample buffer (20µL, with a final concentration of 5% 2-mercaptoethanol) were added to the magnetic particles and heated at 95°C for 5 minutes to obtain a sample for electrophoresis. WB was performed using the simple Western system Wes (Protein Simple). For comparison, the synthetic proIAPP protein was also detected by the WB method. As the primary antibody, the anti-amylin antibody (1:250 dilution; sc-377530) was used, and as the secondary antibody, the Anti-Mouse Secondary Antibody (Protein Simple) was used. The results are shown in FIGS. 5A and 5B.

### (2) Results

As shown in FIG. 5A, a band to which the anti-proIAPP antibody bound was observed in the patient sample (see "Patient" in FIG. 5A). However, no band was observed in the healthy subject sample (see "Healthy" in FIG. 5A). As shown in FIG. 5B, a band to which the anti-proIAPP antibody bound was observed at the same position as the synthetic proIAPP protein in the patient sample (see "Patient" and "Synthetic" in FIG. 5B). These results showed that proIAPP in blood samples can be detected by immunoprecipitation and the WB method. Furthermore, it was shown that patients and healthy subjects can be distinguished by immunoprecipitation and the WB method.

### Example 4: Measurement of proIAPP in blood sample by ELISA method

It was examined whether proIAPP in the plasma of the patients and healthy subjects in Example 3 could be detected by the ELISA method. For comparison, IAPP in the plasma was also measured.

### (1) ELISA Method

### (1.1) Measurement of proIAPP

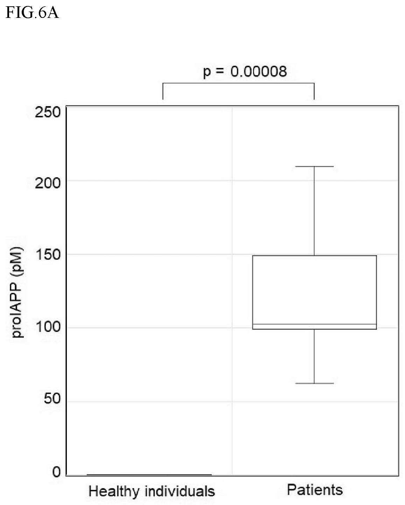

The anti-proIAPP N-terminal antibody was diluted with 25 mM MES buffer (pH6) to prepare a 3.64µg/mL capture antibody solution. 100µL of the obtained antibody solution was dispensed into each well of a 96-well white plate (Thermo Fisher Scientific) and left to stand overnight at 4°C. The wells were washed 3 times with HISCL washing solution (400µL; Sysmex Corporation). 350µL of blocking buffer was dispensed into each well and left to stand for 1 hour at room temperature. The wells were washed 3 times with HISCL washing solution (400µL). 66µL of reaction buffer was dispensed into each well, and 33µL of the specimen was further added to each well. The plate was shaken at 600 rpm for 4 hours at room temperature using a shaker. The wells were washed 3 times with HISCL washing solution (400µL; Sysmex Corporation). The anti-amylin antibody (sc-377530) was diluted 1:1000 with Reagent Diluent Concentrate 1 (DY007B; R&D systems) to obtain the detection antibody solution. 100µL of the obtained antibody solution was dispensed into each well, and the plate was shaken at 600 rpm for 1 hour at room temperature using a shaker. The wells were washed 3 times with HISCL washing solution (400µL). The wells were washed 1 time with washing solution (400µL; DY007B; R&D systems). Anti-Mouse IgG-HRP antibody (330; MBL) was diluted 1:10000 with Reagent Diluent Concentrate 1 to obtain the secondary antibody solution. 100µL of the obtained antibody solution was dispensed into each well, and the plate was shaken at 600 rpm for 1 hour at room temperature using a shaker. The wells were washed 6 times with washing solution (400µL; R&D systems). 100µL of chemiluminescent substrate (293-78804; Fujifilm Wako Pure Chemical Corporation) was dispensed into each well and incubated for 10 minutes at room temperature. The plate was set on a microplate reader, and the luminescence intensity was measured. For the creation of a calibration curve, calibration curve samples prepared by diluting the synthetic proIAPP protein with sample diluent (CS617657, Sysmex Corporation) were measured in the same manner as above. The Mann-Whitney U test was used to determine the statistical significance between the patient group and the healthy subject group. A significant difference was determined when the p-value was less than 0.05. The results are shown in FIG. 6A.

### (1.2) Measurement of IAPP

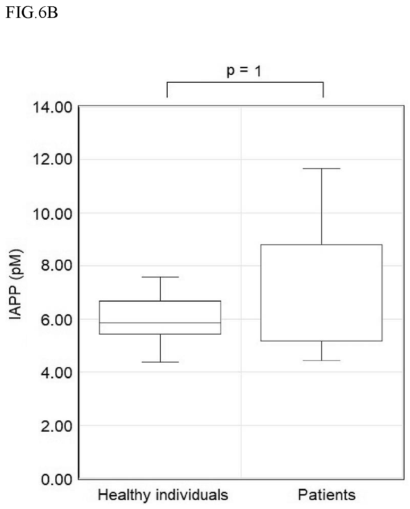

IAPP in the plasma of the patients and healthy subjects was measured using the ELISA Kit for Amylin (CEA812Hu; Cloud-Clone). The specific procedure followed the protocol included with the kit. The statistical significance between the patient group and the healthy subject group was determined by the Mann-Whitney U test. A significant difference was determined when the p-value was less than 0.05. The results are shown in FIG. 6B.

### (2) Results

As shown in FIG. 6A, the concentration of proIAPP was significantly higher in the patient group compared to the healthy subject group. On the other hand, as shown in FIG. 6B, there was no significant difference in the concentration of IAPP between the patient group and the healthy subject group. Therefore, it was suggested that proIAPP in plasma can be used as a biomarker that enables the discrimination of proIAPP-type renal amyloidosis. Referring to FIG. 6A, it was shown that a cutoff value for the proIAPP concentration can be set from a range equal to or greater than 26.7 pM and less than 62.5 pM.

### Example 5: Measurement of proIAPP in blood sample by ELISA method (2)

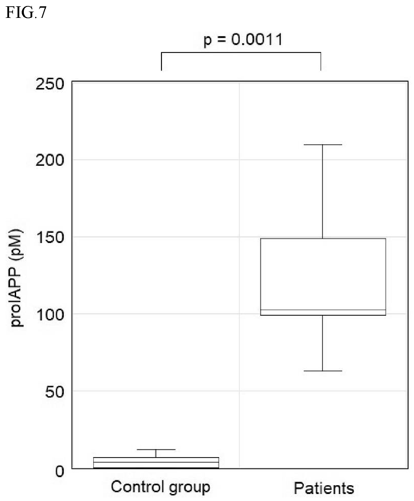

It was examined whether the patients in Example 3 could be distinguished from the disease control group (control 1 to control 13) based on the measured plasma proIAPP values obtained by the ELISA method. The disease control group consisted of patients with the diseases shown in Table 2. These diseases were renal diseases different from proIAPP-type renal amyloidosis. For reference, the eGFR values of the patients in Example 3 are shown in Table 3. The measurement of proIAPP in the blood samples by the ELISA method was performed in the same manner as in Example 4. The statistical significance between the patient group and the disease control group was determined by the Mann-Whitney U test. A significant difference was determined when the p-value was less than 0.05. The results are shown in FIG. 7.

**[Table 2]**

| No. | Disease | GFR (mL/min/1.73 m²) |
|---|---|---|
| control 1 | Focal segmental glomerular sclerosis (FSGS) | 63 |
| control 2 | Chronic Pyelonephritis | 52 |
| control 3 | Focal segmental glomerular sclerosis (FSGS) | 40 |
| control 4 | Tubulointerstitial nephritis | 45 |
| control 5 | Tubulointerstitial nephritis | 60 |
| control 6 | Focal segmental glomerular sclerosis (FSGS) | 39 |
| control 7 | Chronic Pyelonephritis | 40 |
| control 8 | Tubulointerstitial nephritis | 55 |
| control 9 | Focal segmental glomerular sclerosis (FSGS) | 72 |
| control 10 | Tubulointerstitial nephritis | 63 |
| control 11 | Focal segmental glomerular sclerosis (FSGS) | 48 |
| control 12 | Focal segmental glomerular sclerosis (FSGS) | 42 |
| control 13 | Chronic Pyelonephritis | 48 |

**[Table 3]**

| No. | eGFR (mL/min/1.73 m²) |
|---|---|
| Patient 1 | 77 |
| Patient 2 | 64 |
| Patient 3 | 47 |
| Patient 4 | 62 |
| Patient 5 | 51 |

As shown in FIG. 7, the concentration of proIAPP was significantly higher in the patient group compared to the disease control group. Therefore, it was suggested that proIAPP in plasma is a biomarker that can distinguish proIAPP-type renal amyloidosis from other renal diseases. Referring to FIG. 7, it was shown that a cutoff value for the proIAPP concentration can be set from a range equal to or greater than 27.5 pM and less than 62.5 pM.

### [Description of Reference Numerals]

11 Reagent kit
12 first container
13 second container
14 packaging box
15 package insert

## Claims

1. A method for obtaining information on renal amyloidosis, comprising a step of measuring proIAPP in a biological sample from a subject, wherein a result of the measuring is an indicator of proIAPP-type renal amyloidosis.

2. The method according to claim 1, wherein when a measured value of proIAPP is equal to or greater than a threshold, it is suggested that the subject has proIAPP-type renal amyloidosis, and when the measured value of proIAPP is less than the threshold, it is suggested that the subject does not have proIAPP-type renal amyloidosis.

3. The method according to claim 1, wherein the step of measuring proIAPP is performed by an immunological assay or mass spectrometry.

4. The method according to claim 1, wherein the subject is a subject having a urine protein/creatinine (Cr) ratio of 0.15 g/gCr or more, a subject having a urine albumin/Cr ratio of 30 mg/gCr or more, a subject having a urine protein concentration of 30 mg/dL or more, a subject having an eGFR of 90 mL/min/1.73 m² or less, or a subject having a GFR of 90 mL/min/1.73 m² or less.

5. The method according to claim 1, wherein the subject is a patient with chronic kidney disease.

6. The method according to claim 1, wherein the subject is a patient with diabetic nephropathy.

7. The method according to claim 1, wherein the subject is a patient with nephrotic syndrome.

8. The method according to claim 1, wherein the biological sample is a blood sample, or tissue or cells collected by biopsy.

9. A method for assisting diagnosis of renal amyloidosis, comprising steps of:
measuring proIAPP in a biological sample from a subject; and
determining, based on a result of the measuring, whether the subject has proIAPP-type renal amyloidosis.

10. The method according to claim 9, wherein when a measured value of proIAPP is equal to or greater than a threshold, the subject is determined to have proIAPP-type renal amyloidosis, and when the measured value of proIAPP is less than the threshold, the subject is determined not to have proIAPP-type renal amyloidosis.

11. A reagent kit for use in the method according to any one of claims 1 to 10, comprising a reagent comprising a substance capable of specifically binding to proIAPP.

12. The reagent kit according to claim 11, wherein the substance capable of specifically binding to proIAPP is an antibody.
